# EUROPEAN PATENT APPLICATION

(11) **EP 4 560 341 A1**
(43) Date of publication of application: **28.05.2025**
(21) Application number: 24214293.3
(22) Date of filing: 20.11.2024
(51) Int. Cl.: G01R 33/34, G01R 33/3415, G01R 33/36

(54) **MAGNETIC RESONANCE IMAGING APPARATUS**

(30) Priority: 21.11.2023 JP 2023197206; 03.10.2024 JP 2024174100
(71) Applicant: Canon Medical Systems Corporation, Tochigi 324-0036 (JP)
(72) Inventor: OKAMOTO, Kazuya, Otawara-shi (JP); OZAKI, Masanori, Otawara-shi (JP)
(74) Representative: Marks & Clerk LLP

(57) **Abstract**

A magnetic resonance imaging apparatus (100) according to an embodiment includes a receiver coil (106, 107) that receives a magnetic resonance signal, and a couchtop (115) that a subject is placed on and that moves the subject to an imaging region. The couchtop (115) has a space to stow the receiver coil (106, 107) in one or more portions of: widthwise side portions of the couchtop (115); lengthwise end portions of the couchtop (115); and a bottom portion of the couchtop (115). The receiver coil (106, 107) is configured to be used by being pulled out and/or pushed out from the one or more portions of the couchtop (115).

## Description

### FIELD

Embodiments disclosed in the specification and drawings are related to a magnetic resonance imaging apparatus.

### BACKGROUND

Magnetic resonance imaging (MRI) apparatuses conventionally implement imaging of a desired cross section by application of a gradient magnetic field and a radio frequency magnetic field of a magnetic resonance frequency to a subject in a static magnetic field.

When imaging of a body portion of a subject is performed by use of such an MRI apparatus, for example, a spine receiver coil to receive a signal from the back of the subject is placed on a couchtop that moves the subject to an imaging region, the subject is laid thereon, a body receiver coil is then placed on the subject thereafter, and the imaging is performed.

Generally, the spine receiver coil is constantly placed on the couchtop, but the body receiver coil is put at a place for storage, for example, on a shelf, is carried to the couchtop where the subject is lying on from the place for storage, and is then placed on the subject. In a case where the range of the imaging is wide, two or more body receiver coils may be used. Therefore, carrying such receiver coils and placing them on a subject every time imaging is performed have been troublesome work.

There is thus a demand for facilitating placement of receiver coils for MRI apparatuses on subjects.

### SUMMARY OF INVENTION

A magnetic resonance imaging apparatus, comprising:
a receiver coil that receives a magnetic resonance signal; and
a couchtop that a subject is placed on and that moves the subject to an imaging region, wherein
the couchtop has a space to stow the receiver coil in one or more portions of: widthwise side portions of the couchtop; lengthwise end portions of the couchtop; and a bottom portion of the couchtop, and
the receiver coil is configured to be used by being pulled out and/or pushed out from the one or more portions of the couchtop.

The couchtop may have a couchtop frame that supports a load on the couchtop and that is movable to the imaging region, and
the couchtop frame may haves an upper surface where the subject is to be placed on and the couchtop frame may form the space in the one or more portions of the couchtop.

The couchtop frame may have a couchtop supporting portion that supports the upper surface, and
the couchtop supporting portion may have a column-shaped or wall-shaped structure.

The receiver coil may be a body receiver coil that receives a magnetic resonance signal from a body portion of the subject.

The receiver coil may be a head receiver coil that receives a magnetic resonance signal from the head of the subject.

The receiver coil may be configured to be placed on the subject by being pulled out and/or pushed out from at least one of the widthwise side portions of the couchtop or at least one of the lengthwise end portions of the couchtop.

The space may accommodate a plurality of the receiver coils arranged to be lined up in a direction of movement of the couchtop.

The magnetic resonance imaging apparatus may further comprise, separately from the receiver coil to be stowed in the space, a spine receiver coil that receives a magnetic resonance signal from the back of the subject.

The couchtop may have the space to stow the receiver coil in both of the widthwise side portions of the couchtop, and
the receiver coil may have a first receiver coil portion and a second receiver coil portion, the first receiver coil portion being configured to be placed on the subject by being pulled out and/or pushed out from one of the widthwise side portions of the couchtop, the second receiver coil portion being configured to be placed on the subject by being pulled out and/or pushed out from the other one of the widthwise side portions of the couchtop.

The first receiver coil portion and the second receiver coil portion may be configured such that an end portion of the first receiver coil portion and an end portion of the second receiver coil portion are connectible to each other on the subject.

The first receiver coil portion may include a first coil element arranged near the end portion to be connected to the second receiver coil portion,
the second receiver coil portion may include a second coil element arranged near the end portion to be connected to the first receiver coil portion, and
at least the first receiver coil portion and the second receiver coil portion may have a decoupling structure that implements decoupling between the first coil elements and the second coil elements when the end portion of the first receiver coil portion and the end portion of the second receiver coil portion are connected to each other.

At least the first receiver coil portion and the second receiver coil portion may have, as the decoupling structure: a structure having part of the first coil elements and part of the second coil elements overlapping each other when the end portion of the first receiver coil portion and the end portion of the second receiver coil portion are connected to each other; and/or decoupling circuitry that is arranged between the first coil elements and the second coil elements when the end portion of the first receiver coil portion and the end portion of the second receiver coil portion are connected to each other.

At least one of the first receiver coil portion and the second receiver coil portion may have a positioning structure that positions a connection position of the end portions of the first and second receiver coil portions to a position where decoupling by the decoupling structure is possible.

At least one of the first receiver coil portion and the second receiver coil portion may have, as the positioning structure, a non-magnetic fixation tool attached to the end portion of the at least one of the first receiver coil portion and the second receiver coil portion.

The couchtop may have the space to stow the receiver coil in one of the widthwise side portions of the couchtop or one of the lengthwise end portions of the couchtop, and
the receiver coil may be configured to be pulled out and/or pushed out from the one of the widthwise side portions of the couchtop or the one of the lengthwise end portions of the couchtop and to be connected and fixed to the other one of the widthwise side portions of the couchtop or the other one of the lengthwise end portions of the couchtop.

### BRIEF DESCRIPTION OF THE DRAWINGS

FIG. 1 is a diagram illustrating an example of a configuration of an MRI apparatus according to a first embodiment;
FIG. 2 is a sectional view of an example of a couchtop and a body receiver RF coil, according to the first embodiment, the sectional view illustrating a state where the coil has been stowed;
FIG. 3 is a top view of an example of the couchtop and body receiver RF coils, according to the first embodiment, the top view illustrating a state where the coils have been stowed;
FIG. 4 is a sectional view of the example of the couchtop and the body receiver RF coil, according to the first embodiment, the sectional view illustrating a state where the coil has been pulled out and/or pushed out;
FIG. 5 is a top view of the example of the couchtop and the body receiver RF coils, according to the first embodiment, the top view illustrating a state where the coils have been pulled out and/or pushed out;
FIG. 6 is a perspective view of the example of the couchtop and the body receiver RF coils, according to the first embodiment, the perspective view illustrating the state where the coils have been pulled out and/or pushed out;
FIG. 7A to FIG. 7C are diagrams illustrating examples of a method of connecting receiver RF coil portions included in a body receiver RF coil according to the first embodiment and a method of decoupling between coil elements;
FIG. 8A to FIG. 8C are diagrams illustrating examples of a coil holding member included in the body receiver RF coil according to the first embodiment; and
FIG. 9A and FIG. 9B are sectional views of an example of a couchtop and a body receiver RF coil according to a second embodiment.

### DETAILED DESCRIPTION

An MRI apparatus according to an embodiment includes a receiver coil that receives a magnetic resonance signal, and a couchtop that a subject is placed on and that moves the subject to an imaging region. The couchtop has a space to stow the receiver coil in any one or more portions of: widthwise side portions of the couchtop; lengthwise end portions of the couchtop; and a bottom portion of the couchtop. The receiver coil is configured to be used by being pulled out and/or pushed out from the one or more portions of the couchtop.

Embodiments of an MRI apparatus according to the present application will hereinafter be described in detail while reference is made to the drawings.

### First Embodiment

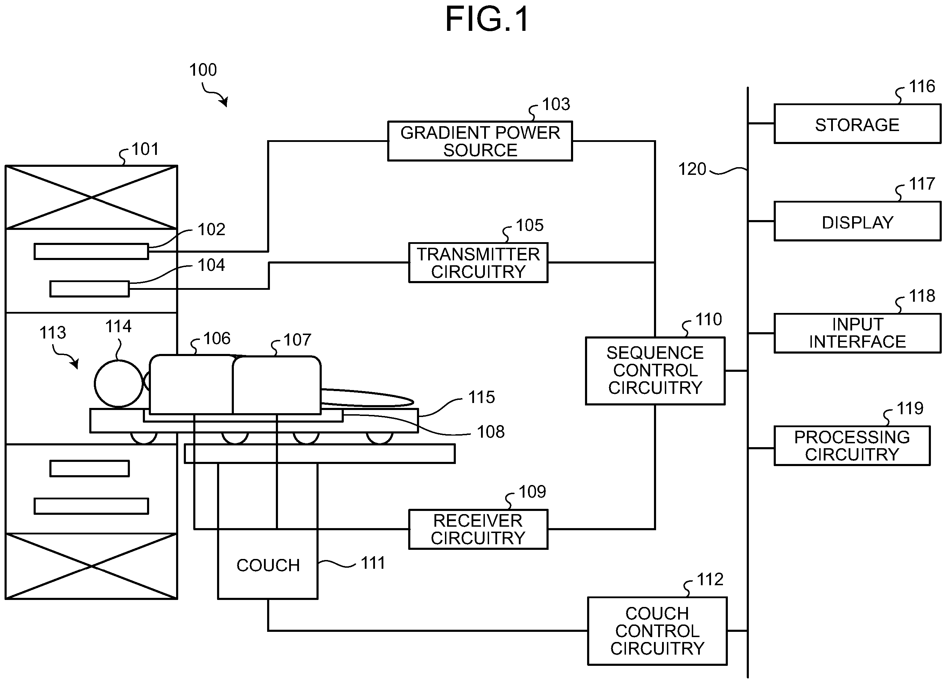

FIG. 1 is a diagram illustrating an example of a configuration of an MRI apparatus according to a first embodiment.

As illustrated in FIG. 1, for example, an MRI apparatus 100 according to this embodiment includes: a static magnetic field magnet 101; a gradient coil 102; a gradient power source 103; a couch 111; couch control circuitry 112; a transmitter RF coil 104; transmitter circuitry 105; a first body receiver RF coil 106; a second body receiver RF coil 107; a spine receiver RF coil 108; receiver circuitry 109; sequence control circuitry 110; a bus 120; an input interface 118; a display 117; a storage 116; and processing circuitry 119. The MRI apparatus 100 may have a hollow cylindrical shim coil between the static magnetic field magnet 101 and the gradient coil 102.

The static magnetic field magnet 101 is a magnet formed in a hollow cylindrical shape and generates a uniform static magnetic field (B0) in a space inside the static magnetic field magnet 101. For example, a superconducting magnet is used as this static magnetic field magnet 101. A shim coil not illustrated in the drawings may be formed in a hollow cylindrical shape inside the static magnetic field magnet 101. The shim coil is connected to a shim coil power source not illustrated in the drawings, and uniformizes a static magnetic field generated by the static magnetic field magnet 101 by means of electric power supplied from the shim coil power source.

The gradient coil 102 is a coil formed in a hollow cylindrical shape and is arranged inside the static magnetic field magnet 101. The gradient coil 102 is formed of a combination of three coils respectively corresponding to an X-axis , a Y-axis, and a Z-axis orthogonal to one another. The direction of the Z-axis is the same as the direction of the static magnetic field. The direction of the Y-axis is a vertical direction and the direction of the X-axis is a direction perpendicular to the Z-axis and the Y-axis. The three coils in the gradient coil 102 generate gradient magnetic fields that respectively change in magnetic field strength along the X-axis, Y-axis, and Z-axis by individually receiving supply of electric current from the gradient power source 103.

The gradient magnetic fields along the X-axis, Y-axis, and Z-axis generated by the gradient coil 102 respectively correspond to, for example, a frequency encoding gradient magnetic field (also referred to as a readout gradient magnetic field), a phase encoding gradient magnetic field, and a slice selection gradient magnetic field. The frequency encoding gradient magnetic field is used for changing the frequency of a magnetic resonance (MR) signal according to a spatial position. The phase encoding gradient magnetic field is used for changing the phase of the MR signal according to the spatial position. The slice selection gradient magnetic field is used for optionally determining an imaging cross section.

The gradient power source 103 is a power source device that supplies electric current to the gradient coil 102, under control by the sequence control circuitry 110.

The couch 111 is an apparatus including a couchtop 115 where a subject 114 is placed on. The couch 111 inserts the couchtop 115 where the subject 114 has been placed, into a bore 113, under control by the couch control circuitry 112. The couchtop 115 thereby moves the subject 114 to an imaging region in the bore 113. Normally, the couch 111 is installed in an examination room where the MRI apparatus 100 has been installed, such that a longitudinal direction of the couch 111 is parallel to a central axis of the static magnetic field magnet 101.

The couch control circuitry 112 controls the couch 111. The couch control circuitry 112 drives the couch 111 according to instructions input by an operator via the input interface 118 and thereby moves the couchtop 115 in the longitudinal direction and upward and downward directions.

The transmitter RF coil 104 is a radio frequency (RF) coil placed inside the gradient coil 102. The transmitter RF coil 104 generates transmission RF waves corresponding to a radio frequency magnetic field by receiving supply of radio frequency pulses (RF pulses) from the transmitter circuitry 105. The transmitter RF coil 104 is, for example, a whole body (WB) coil. The WB coil may be used as a transmitter-receiver RF coil.

Under control by the sequence control circuitry 110, the transmitter circuitry 105 supplies radio frequency pulses modulated with the Larmor frequency. Specifically, the transmitter circuitry 105 has an oscillator, a phase selector, a frequency converter, an amplitude modulator, and a radio frequency power amplifier, for example. The oscillator generates a radio frequency signal having a resonance frequency specific to a target atomic nucleus in the static magnetic field. The phase selector selects a phase of the high frequency signal. The frequency converter converts the frequency of the radio frequency signal output from the phase selector. The amplitude modulator modulates the amplitude of the radio frequency signal output from the frequency modulator according to a sinc function, for example. The radio frequency power amplifier amplifies the radio frequency signal output from the amplitude modulator. As a result of operation of these components, the transmitter circuitry 105 outputs the radio frequency pulses corresponding to the Larmor frequency, to the transmitter RF coil 104.

In the example described with respect to this embodiment, three types of receiver RF coils, the first body receiver RF coil 106, the second body receiver RF coil 107, and the spine receiver RF coil 108, are used. The first body receiver RF coil 106 and the second body receiver RF coil 107 are placed on the subject 114. The spine receiver RF coil 108 is placed on the couchtop 115, on a back side of the subject 114.

These receiver RF coils are moved to the imaging region in the bore 113 by the couchtop 115 after being placed on the subject 114 and receive MR signals radiated from the subject 114 due to the radio frequency magnetic field. The first body receiver RF coil 106 and the second body receiver RF coil 107 mainly receive MR signals from a body portion of the subject 114. The spine receiver RF coil 108 mainly receives MR signals from the back of the subject 114. These receiver RF coils then output the received MR signals to the receiver circuitry 109. Typically, these receiver RF coils are each a coil array having plural coil elements and signals from valid coil elements surrounding a region to be imaged are automatically or manually selected.

While the receiver RF coils are being used for imaging, under control by the sequence control circuitry 110, the receiver RF coils are controlled such that the receiver RF coils are: in an off state when radio frequency pulses are being generated by the transmitter RF coil 104; and in an on state where the receiver RF coils are capable of receiving signals, at any other time.

On the basis of the MR signals output from the respective receiver RF coils under control by the sequence control circuitry 110, the receiver circuitry 109 generates magnetic resonance data (hereinafter, referred to as MR data) that are complex number data that have been digitalized. Specifically, after subjecting the MR signals output from the respective receiver RF coils to various kinds of signal processing, such as preamplification, intermediate frequency conversion, phase detection, low frequency amplification, and filtering, the receiver circuitry 109 executes analog to digital (A/D) conversion of data that have been subjected to the various kinds of signal processing. The receiver circuitry 109 thereby generates MR data. The receiver circuitry 109 outputs the MR data generated, to the sequence control circuitry 110. The MR data generated by the receiver circuitry 109 are also called raw data.

According to pulse sequence information output from the processing circuitry 119, the sequence control circuitry 110 controls the gradient power source 103, the transmitter circuitry 105, and the receiver circuitry 109, to perform imaging of the subject 114. The pulse sequence information includes, for example, a level and a time width of electric current supplied to the gradient coil 102 by the gradient power source 103, timing of supply of the electric current to the gradient coil 102 by the gradient power source 103, an amplitude of an RF pulse supplied to the transmitter RF coil 104 by the transmitter circuitry 105, timing of supply of the RF pulse to the transmitter RF coil 104 by the transmitter circuitry 105, and timing of reception of an MR signal by the receiver circuitry 109. The level of the electric current supplied to the gradient coil 102 by the gradient power source 103 corresponds to a waveform of the gradient magnetic field corresponding to a pulse sequence.

The bus 120 is a transmission line where data are transmitted through between the input interface 118, the display 117, the storage 116, and the processing circuitry 119. Various biological signal measurement devices and an external storage device may be appropriately connected to the bus 120 via a network, for example.

The input interface 118 receives various instructions and input of information, from an operator. The input interface 118 is, for example, circuitry related to an input device that may be a pointing device, such as a mouse, or a keyboard. The input interface 118 is not limited to circuitry related to a physical operating component, such as a mouse or a keyboard. Examples of the input interface 118 may include electric signal processing circuitry that receives electric signals corresponding to input operation from an external input device provided separately from the MRI apparatus 100 and outputs the received electric signals to various kinds of circuitry.

Under control by the processing circuitry 119, the display 117 displays various kinds of information, such as MR images that have been reconstructed by an image generating function. The display 117 is, for example, a display device, such as a cathode ray tube (CRT) display, a liquid crystal display, an organic electroluminescence (EL) display, a light emitting diode (LED) display, a plasma display, or any other display or monitor known in the art.

The storage 116 stores therein, for example, MR data arranged in a k-space via a data arranging function and image data generated by the image generating function. The storage 116 stores therein, for example, imaging conditions including various imaging protocols and plural imaging parameters prescribing the imaging protocols. The storage 116 stores programs corresponding to various functions executed by the processing circuitry 119. The storage 116 is, for example: a semiconductor memory element, such as a random access memory (RAM) or a flash memory; a hard disk drive; a solid state drive; or an optical disk. The storage 116 may be, for example, a drive device that reads and writes various kinds of information from and in a portable storage medium, such as a compact disk read only memory (CD-ROM) drive, a digital versatile disk (DVD) drive, or a flash memory.

The processing circuitry 119 integrally controls the MRI apparatus 100. The processing circuitry 119 is implemented by, for example, a processor. The processing circuitry 119 has a system controlling function, the data arranging function, the image generating function, a reference value setting function, an error estimating function, a correcting function, and a pulse calculating function. Various functions implemented by the system controlling function, the data arranging function, the image generating function, the reference value setting function, the error estimating function, the correcting function, and the pulse calculating function have been stored in the storage 116, in a form of programs executable by a computer. By reading and executing the programs corresponding to these various functions from the storage 116, the processing circuitry 119 implements the functions corresponding to these programs.

Specifically, the processing circuitry 119 integrally controls the MRI apparatus 100 by means of the system controlling function. For example, the processing circuitry 119 reads a system control program stored in the storage 116, loads the system control program into a memory, and controls the respective pieces of circuitry of the MRI apparatus 100 according to the system control program loaded.

The various functions have been described as being implemented by the single piece of processing circuitry 119 with respect to FIG. 1, but the processing circuitry 119 may be formed of a combination of plural independent processors and the functions may be implemented by these processors executing the programs. In other words, each of the above mentioned functions may be configured as a program and a single piece of processing circuitry may execute each program, or a specific function may be implemented by a piece of dedicated independent program execution circuitry. Furthermore, in the example of the case described by reference to FIG. 1, the single storage 116 stores the programs corresponding to the functions but the embodiment is not limited to this example. For example, plural storages may be arranged in a distributed manner and the processing circuitry 119 may be configured to read and execute the corresponding programs from the individual storages.

Furthermore, the couch control circuitry 112, the transmitter circuitry 105, the receiver circuitry 109, and the sequence control circuitry 110 are similarly formed of processing circuitry, such as a processor, for example.

The term, "processor", used in the description above means, for example: a central processing unit (CPU); a graphics processing unit (GPU); or a circuit, such as an application specific integrated circuit (ASIC) or a programmable logic device (for example, a simple programmable logic device (SPLD), a complex programmable logic device (CPLD), or a field programmable gate array (FPGA)).

The processor implements the various functions by reading and executing the programs stored in the storage 116. Instead of being stored in the storage 116, the programs may be directly incorporated in circuitry of the processor. In this case, by reading and executing the programs incorporated in the circuitry, the processor implements the functions.

The MRI apparatus 100 according to the embodiment is thus configured to facilitate placement of a body receiver RF coil on the subject 114.

Specifically, in this embodiment, the couchtop 115 of the couch 111 has a space to stow a body receiver RF coil in widthwise side portions (hereinafter, referred to as the "side portions") and a bottom portion of the couchtop 115. The body receiver RF coil is configured to be used by being pulled out from the side portions and bottom portion of the couchtop 115. The body receiver RF coil is an example of a receiver coil.

Furthermore, in this embodiment, the couchtop 115 has a couchtop frame that supports a load on the couchtop 115 and that is movable to the imaging region. The couchtop frame has an upper surface where the subject 114 is to be placed on and the couchtop frame forms the space to stow the body receiver RF coil in the side portions and bottom portion of the couchtop 115.

Furthermore, in this embodiment, the body receiver RF coil is configured to be placed on the subject 114 by being pulled out and/or pushed out from at least one of the side portions of the couchtop 115.

Furthermore, in this embodiment, the space in the couchtop 115 accommodates a plurality of the body receiver RF coils arranged to be lined up in a direction of movement of the couchtop 115.

The configuration of the MRI apparatus 100 according to the embodiment will hereinafter be described in more detail.

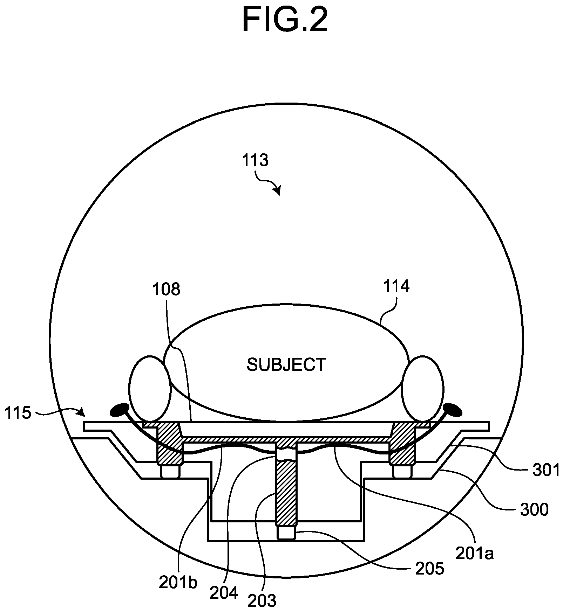

FIG. 2 is a sectional view of an example of the couchtop 115 and a body receiver RF coil, according to the first embodiment, the sectional view illustrating a state where the coil has been stowed.

For example, as illustrated in FIG. 2, the couchtop 115 includes a couchtop frame 203 provided with plural couchtop wheels 205, and a couchtop bottom cover 301 that covers side portions and a bottom portion of the couchtop frame 203. The couchtop frame 203 is configured to support a load on the couchtop 115 and to be movable to the imaging region in the bore 113. Furthermore, the couchtop frame 203 forms the space to stow the body receiver RF coil in the side portions and bottom portion of the couchtop 115 and has the couchtop bottom cover 301 arranged to cover the space. The spine receiver RF coil 108 is arranged on an upper surface of the couchtop frame 203 and the subject 114 is laid thereon. The couchtop wheels 205 move on rails 300 in the bore 113 when the couchtop 115 moves in the bore 113.

For example, the body receiver RF coil (the first body receiver RF coil 106 or the second body receiver RF coil 107) includes two receiver RF coil portions 201a and 201b and a coil holding member 204. One of end portions of the receiver RF coil portion 201a and one of end portions of the receiver RF coil portion 201b have been connected to each other by the coil holding member 204 at the center of the couchtop 115, and when the body receiver RF coil is not being used, the two receiver RF coil portions 201a and 201b are stowed in the space, which has been formed in the bottom portion and side portions of the couchtop 115 by the couchtop frame 203 in the couchtop bottom cover 301. The receiver RF coil portions 201a and 201b are an example of receiver coil portions.

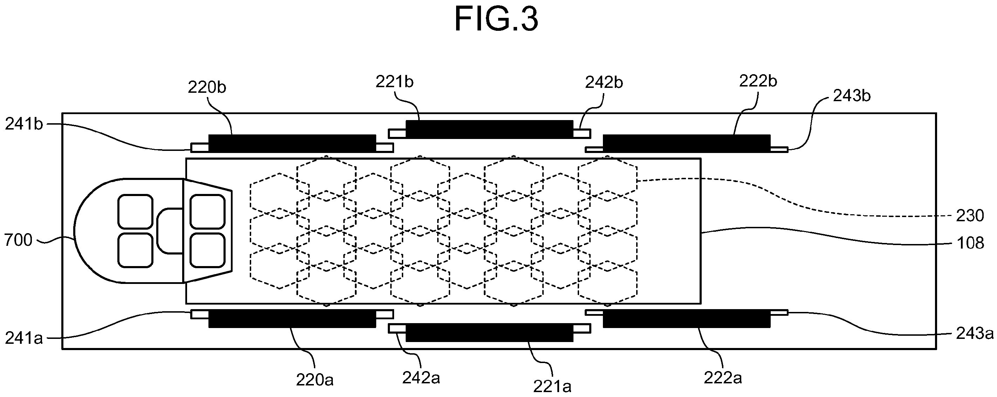

FIG. 3 is a top view of an example of the couchtop 115 and body receiver RF coils, according to the first embodiment, the sectional view illustrating a state where the coils have been stowed. Specifically, FIG. 3 illustrates an example where three body receiver RF coils have been arranged to be lined up in a body axis direction of the subject 114, with the subject 114 having been excluded from the state illustrated in FIG. 2. The body axis direction of the subject 114 is in line with the direction of movement of the couchtop 115.

For example, as illustrated in FIG. 3, a first body receiver RF coil has two receiver RF coil portions 220a and 220b. A second body receiver RF coil has two receiver RF coil portions 221a and 221b. A third body receiver RF coil has two receiver RF coil portions 222a and 222b. The spine receiver RF coil 108 and a head receiver RF coil 700 are also arranged on the upper surface of the couchtop frame 203. The spine receiver RF coil 108 includes plural coil elements 230.

For example, the receiver RF coil portions 220a and 220b of the first body receiver RF coil are respectively stowed in the space in the couchtop bottom cover 301 through coil long holes 241a and 241b provided on an upper surface of the couchtop bottom cover 301. Similarly, the receiver RF coil portions 221a and 221b of the second body receiver RF coil are respectively stowed in the space in the couchtop bottom cover 301 through coil long holes 242a and 242b provided on the upper surface of the couchtop bottom cover 301. Similarly, the receiver RF coil portions 222a and 222b of the third body receiver RF coil are respectively stowed in the space in the couchtop bottom cover 301 through coil long holes 243a and 243b provided on the upper surface of the couchtop bottom cover 301.

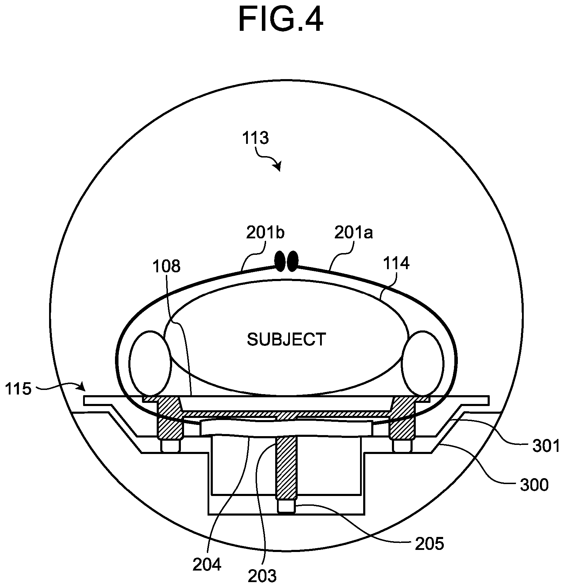

FIG. 4 is a sectional view of the example of the couchtop 115 and the body receiver RF coil, according to the first embodiment, the sectional view illustrating a state where the coil has been pulled out or pushed out. Specifically, FIG. 4 illustrates how the body receiver RF coil has been pulled out and/or pushed out from the state illustrated in FIG. 2 and placed on the subject 114.

For example, as illustrated in FIG. 4, the body receiver RF coil (the first body receiver RF coil 106 or the second body receiver RF coil 107) is placed on the subject 114 by being pulled out and/or pushed out from the side portions of the couchtop 115 when the body receiver RF coil is used in imaging. The body receiver RF coil is configured to enable the two receiver RF coil portions 201a and 201b to be connected to each other on the subject 114 by the coil holding member 204 being extended.

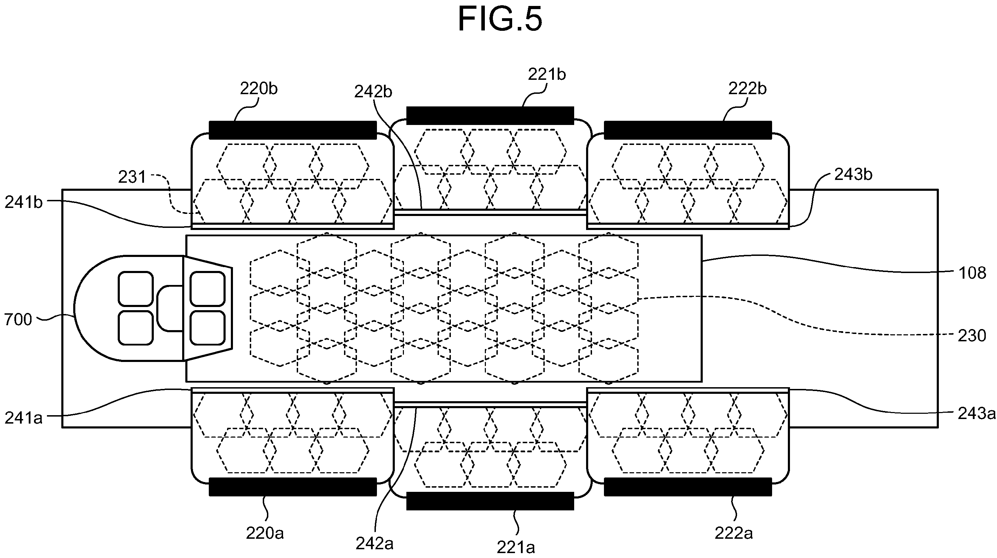

FIG. 5 is a top view of the example of the couchtop 115 and the body receiver RF coils, according to the first embodiment, the top view illustrating a state where the coils have been pulled out and/or pushed out. Specifically, FIG. 5 illustrates how the three body receiver RF coils arranged to be lined up in the body axis direction of the subject 114 have been pulled out and/or pushed out, from the state illustrated in FIG. 3.

For example, as illustrated in FIG. 5, the first body receiver RF coil, the second body receiver RF coil, and the third body receiver RF coil each include plural coil elements 231.

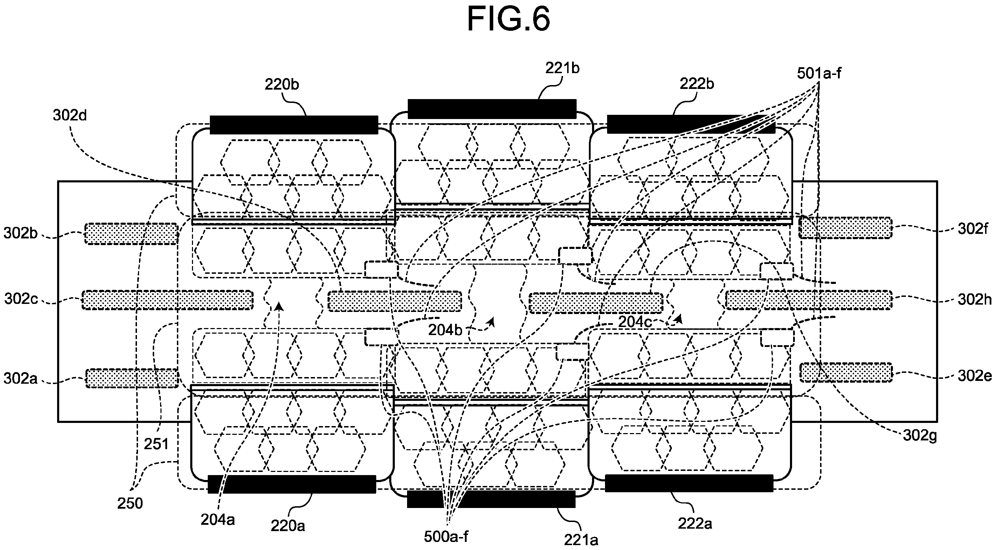

FIG. 6 is a perspective view of the example of the couchtop 115 and the body receiver RF coils, according to the first embodiment, the perspective view illustrating the state where the coils have been pulled out and/or pushed out. Specifically, FIG. 6 illustrates a state where the bottom portion of the couchtop 115 is perspectively viewed, with the spine receiver RF coil 108 and head receiver RF coil 700 removed from the state illustrated in FIG. 5, in which the spine receiver RF coil 108 and head receiver RF coil 700 have been arranged on the upper surface of the couchtop frame 203.

For example, as illustrated in FIG. 6, the couchtop frame 203 has plural couchtop supporting portions 302a to 302h. The couchtop supporting portions 302a to 302h are each provided with a couchtop wheel 205 and has a role of supporting a load on the couchtop 115 and moving the couchtop 115 to the imaging region.

For example, the couchtop supporting portions 302a to 302h each have a column shaped or wall shaped structure and support the upper surface of the couchtop frame 203, the upper surface being where the subject 114 is to be placed.

For example, the couchtop frame 203 has: plural couchtop supporting portions arranged at one of the side portions of the couchtop 115; plural couchtop supporting portions arranged at the other one of the side portions of the couchtop 115; and plural couchtop supporting portions arranged at a widthwise center portion of the couchtop 115, over approximately the whole length of the couchtop 115. The couchtop frame 203 is configured such that the number of couchtop supporting portions arranged at the widthwise center portion of the couchtop 115 is more than the number of the couchtop supporting portions arranged at the one of the side portions of the couchtop 115 and the number of the couchtop supporting portions arranged at the other one of the side portions of the couchtop 115.

For example, as illustrated in FIG. 6, the couchtop frame 203 has the two couchtop supporting portions 302a and 302e arranged at the one of the side portions of the couchtop 115, and the two couchtop supporting portions 302b and 302f arranged at the other one of the side portions of the couchtop 115. The couchtop supporting portions 302a and 302e at the one of the side portions of the couchtop 115 are each arranged outside a range where the receiver RF coil portion 220a of the first body receiver RF coil, the receiver RF coil portion 221a of the second body receiver RF coil, and the receiver RF coil portion 222a of the third body receiver RF coil are arranged along the length of the couchtop 115. Furthermore, the couchtop supporting portions 302b and 302f at the other one of the side portions of the couchtop 115 are each arranged outside a space where the receiver RF coil portion 220b of the first body receiver RF coil, the receiver RF coil portion 221b of the second body receiver RF coil, and the receiver RF coil portion 222b of the third body receiver RF coil are stowed along the length of the couchtop 115. Furthermore, the couchtop frame 203 has the four couchtop supporting portions 302c, 302d, 302g, and 302h arranged at the widthwise center of the couchtop 115. The couchtop supporting portions 302c, 302d, 302g, and 302h are arranged spaced from each other in a line, over approximately the whole length of the couchtop 115.

Generally, a couch of an MRI apparatus is often provided with couchtop supporting portions along widthwise side portions of a couchtop. In contrast, in this embodiment, the space to stow the body receiver RF coils is provided in the side portions of the couchtop 115, the range where the couchtop supporting portions are able to be provided is thus limited in the side portions of the couchtop 115, and one may thus think that strength to support a load from the subject 114, for example, may be reduced. However, according to the above described configuration, a larger number of couchtop supporting portions are provided at the widthwise center portion of the couchtop 115 over approximately the whole length of the couchtop 115 than at each of the side portions of the couchtop 115, and strength to endure the load from the subject 114, for example, is thereby able to be obtained even if the space to stow the body receiver RF coils is provided in the side portions of the couchtop 115.

For example, the receiver RF coil portions 220a and 220b of the first body receiver RF coil are connected to each other by a coil holding member 204a arranged between the two couchtop supporting portions 302c and 302d lined up in the direction of movement of the couchtop 115, the two couchtop supporting portions 302c and 302d being two of the plural couchtop supporting portions 302a to 302h. Similarly, the receiver RF coil portions 221a and 221b of the second body receiver RF coil are connected to each other by a coil holding member 204b arranged between the two couchtop supporting portions 302d and 302g lined up in the direction of movement of the couchtop 115. Similarly, the receiver RF coil portions 222a and 222b of the third body receiver RF coil are connected to each other by a coil holding member 204c arranged between the two couchtop supporting portions 302g and 302h lined up in the direction of movement of the couchtop 115.

Furthermore, for example, as illustrated in FIG. 6, the receiver RF coil portions 220a and 220b of the first body receiver RF coil, the receiver RF coil portions 221a and 221b of the second body receiver RF coil, and the receiver RF coil portions 222a and 222b of the third body receiver RF coil respectively have signal output portions 500a to 500f. The signal output portions 500a to 500f are arranged in the couchtop bottom cover 301 and are respectively connected to the receiver circuitry 109 via cables 501a to 501f arranged in the couchtop bottom cover 301.

Normally, in reception of MR signals for cross sections of the body portion, the spine receiver RF coil 108 and body receiver RF coils are used. In this case, only a body receiver RF coil signal receiving portion 250 of a body receiver RF coil needed for reception of an MR signal is pulled out and/or pushed out, and a body receiver RF coil signal non-receiving portion 251 in a region where the spine receiver RF coil 108 is placed on does not need to receive the MR signal. Therefore, during imaging of the body portion, coil elements 231 included in the body receiver RF coil signal non-receiving portion 251 are in an off state.

Which coil elements 231 are to be brought into the off state is determined by use of, for example, a video captured by a camera to check from outside how much each body receiver RF coil has been pulled out and/or pushed out when that body receiver RF coil has been placed on the subject 114 by being pulled out and/or pushed out, for example. Or the coil elements 231 included in the body receiver RF coil signal non-receiving portion 251 and the coil elements 230 included in the spine receiver RF coil 108 largely change in output impedance by electric coupling between the coil elements 231 and the coil elements 230, and the determination may thus be made by detecting this output impedance. Or the determination may be made by measuring how much the coil holding member 204 is extended for each body receiver RF coil.

As described above, in this embodiment, the couchtop 115 has the space to stow the body receiver RF coil in both of the widthwise side portions of the couchtop 115. Furthermore, the body receiver RF coil has the two receiver RF coil portions 201a and 201b. The receiver RF coil portion 201a is configured to be placed on the subject 114 by being pulled out and/or pushed out from one of the widthwise side portions of the couchtop 115, and the receiver RF coil portion 201b is configured to be placed on the subject 114 by being pulled out and/or pushed out from the other one of the widthwise side portions of the couchtop 115.

Furthermore, in this embodiment, the receiver RF coil portions 201a and 201b are configured such that the end portions of the receiver RF coil portions 201a and 201b are able to be connected to each other on the subject 114.

Any one of various methods may be adopted as a method of connecting the receiver RF coil portion 201a and the receiver RF coil portion 201b to each other. Furthermore, any directly adjacent ones of plural coil elements 231 included in the receiver RF coil portions 201a and 201b are desirably subjected to decoupling when the receiver RF coil portions 201a and 201b are connected to each other. Decoupling means reducing induced electric current generated by one of coil elements arranged adjacently to each other, in the other one of the coil elements.

Specifically, the receiver RF coil portion 201a includes first coil elements arranged near the end portion to be connected to the receiver RF coil portion 201b. Furthermore, the receiver RF coil portion 201b includes second coil elements arranged near the end portion to be connected to the receiver RF coil portion 201a.

At least one of the receiver RF coil portion 201a and the receiver RF coil portion 201b has a decoupling structure that implements decoupling between the first coil elements and the second coil elements when the end portion of the receiver RF coil portion 201a and the end portion of the receiver RF coil portion 201b are connected to each other.

Furthermore, at least one of the receiver RF coil portion 201a and the receiver RF coil portion 201b has a positioning structure enabling positioning of a connection position of the end portions of the receiver RF coil portions 201a and 201b to a position where decoupling by the decoupling structure is possible.

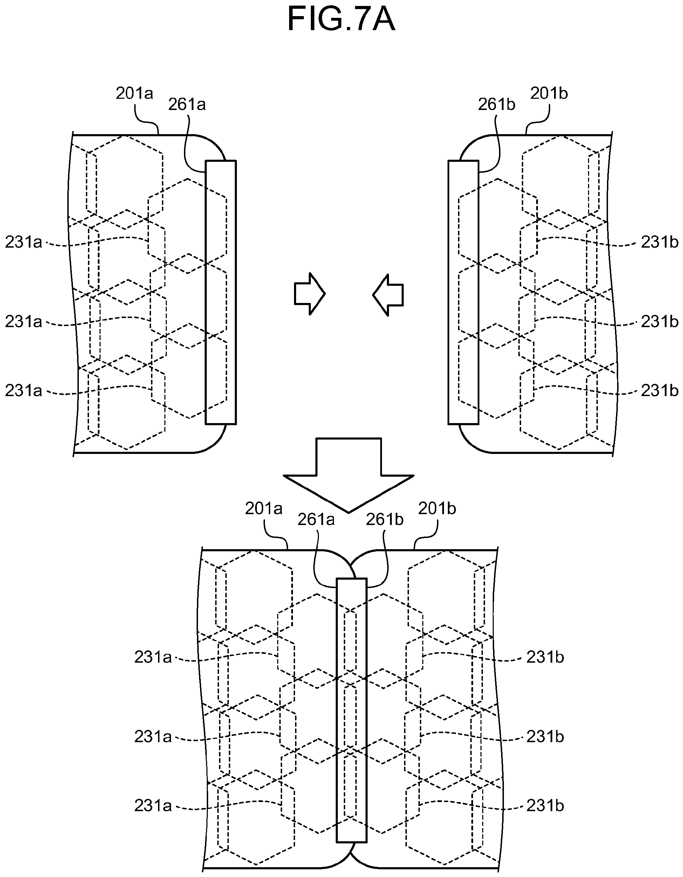

FIG. 7A to FIG. 7C are diagrams illustrating examples of a method of connecting receiver RF coil portions included in a body receiver RF coil and a method of performing decoupling between coil elements, according to the first embodiment.

For example, as illustrated in FIG. 7A, a receiver RF coil portion 201a includes plural coil elements 231a arranged near an end portion to be connected to the receiver RF coil portion 201b. Furthermore, a receiver RF coil portion 201b includes plural coil elements 231b arranged near an end portion to be connected to the receiver RF coil portion 201a.

In the example illustrated in FIG. 7A, the plural coil elements 231a included in the receiver RF coil portion 201a and the plural coil elements 231b included in the receiver RF coil portion 201b are arranged in a width direction of the couchtop 115 to be adjacent to each other when the end portion of the receiver RF coil portion 201a and the end portion of the receiver RF coil portion 201b have been connected to each other.

In this case, for example, at least one of the receiver RF coil portion 201a and the receiver RF coil portion 201b has, as the decoupling structure, a structure having part of the coil elements 231a and part of the coil elements 231b overlapping each other when the end portion of the receiver RF coil portion 201a and the end portion of the receiver RF coil portion 201b have been connected to each other.

For example, as illustrated in FIG. 7A, in a case where the receiver RF coil portion 201a and the receiver RF coil portion 201b are configured to be connected to each other with their end portions overlapping each other, the coil elements 231a and the coil elements 231b are arranged in the receiver RF coil portion 201a and the receiver RF coil portion 201b such that part of the coil elements 231a and part of the coil elements 231b are included in a range where the receiver RF coil portion 201a and the receiver RF coil portion 201b overlap each other.

Or, for example, the coil elements 231a may be formed to have a portion protruding from the end portion of the receiver RF coil portion 201a, and the portion protruding may be configured to overlap part of the coil elements 231b when the end portion of the receiver RF coil portion 201a and the end portion of the receiver RF coil portion 201b have been connected to each other. Similarly, the coil elements 231b may be formed to have a portion protruding from the end portion of the receiver RF coil portion 201b, and the portion protruding may be configured to overlap part of the coil elements 231a when the end portion of the receiver RF coil portion 201a and the end portion of the receiver RF coil portion 201b have been connected to each other.

Furthermore, for example, at least one of the receiver RF coil portion 201a and the receiver RF coil portion 201b has, as the positioning structure, a non-magnetic fixation tool attached to at least one of the end portions thereof.

For example, as illustrated in FIG. 7A, the receiver RF coil portion 201a has, as the nonmagnetic fixation tool, a hook fastener 261a of a hook and loop fastener, such as MAGICTAPE (registered trademark) or VELCRO (registered trademark) and the receiver RF coil portion 201b has, as the nonmagnetic fixation tool, a loop fastener 261b of the hook and loop fastener.

Or, for example, the receiver RF coil portion 201a may have, as the non-magnetic fixation tool, a stud of a plastic button (snap button), and the receiver RF coil portion 201b may have, as the non-magnetic fixation tool, a socket of the plastic button.

Or, for example, any one of the receiver RF coil portion 201a and the receiver RF coil portion 201b may have a fixation tool that is able to be coupled to the other one of the receiver RF coil portion 201a and the receiver RF coil portion 201b itself, like a plastic hook.

Furthermore, for example, one or both of the receiver RF coil portion 201a and the receiver RF coil portion 201b may have a marker indicating a connection position of the end portions of the receiver RF coil portions 201a and 201b, the connection position being where decoupling by the decoupling structure is possible.

Furthermore, in another example, at least one of the receiver RF coil portion 201a and the receiver RF coil portion 201b may have, as the decoupling structure, decoupling circuitry arranged between the coil elements 231a and the coil elements 231b when the end portion of the receiver RF coil portion 201a and the end portion of the receiver RF coil portion 201b have been connected to each other.

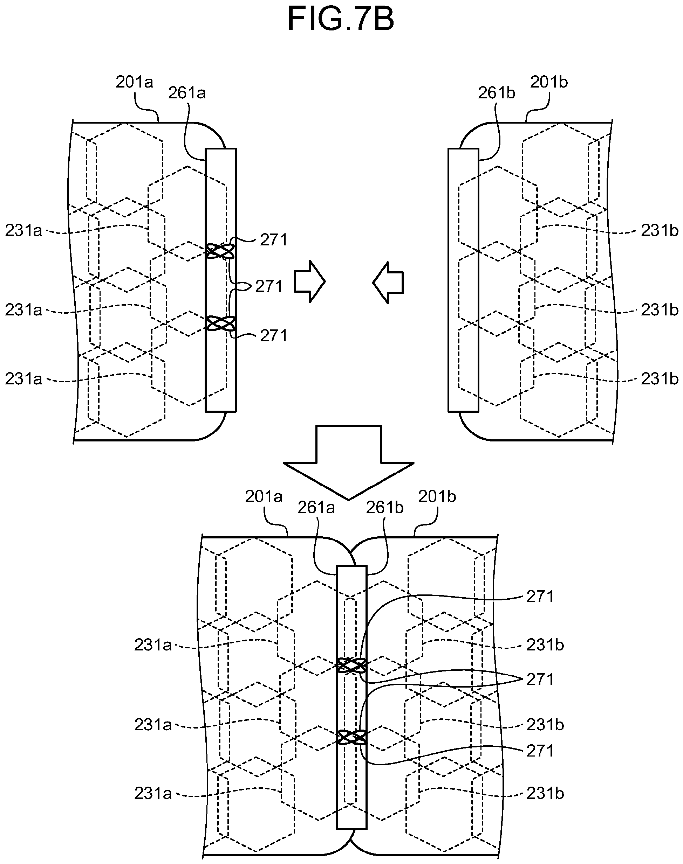

For example, as illustrated in FIG. 7B, the receiver RF coil portion 201a illustrated in FIG. 7A further has decoupling circuitry 271. The decoupling circuitry 271 has been attached to the receiver RF coil portion 201a such that the decoupling circuitry 271 is arranged between the coil elements 231a and the coil elements 231b that are arranged diagonally to the width direction of the couchtop 115 when the end portion of the receiver RF coil portion 201a and the end portion of the receiver RF coil portion 201b have been connected to each other.

For example, the decoupling circuitry 271 may be attached to the receiver RF coil portion 201b or may be attached in a distributed manner to both the receiver RF coil portion 201a and the receiver RF coil portion 201b.

Between the plural coil elements 231a included in the receiver RF coil portion 201a and the plural coil elements 231b included in the receiver RF coil portion 201b, the above described configuration achieves decoupling by overlap between parts of adjacent coil elements, the adjacent coil elements being adjacent ones of the coil elements 231a and 231b and being adjacent to each other in the width direction of the couchtop 115, when the end portion of the receiver RF coil portion 201a and the end portion of the receiver RF coil portion 201b are connected to each other, achieves decoupling by decoupling circuitry for coil elements arranged diagonally to the width direction of the couchtop 115, and thus enables decoupling between the coil elements in a comprehensive manner.

In the example illustrated in FIG. 7B, the receiver RF coil portion 201a and the receiver RF coil portion 201b have both the structure with the parts of the adjacent coil elements overlapping each other and the decoupling circuitry arranged between the adjacent coil elements, but the embodiment is not limited to this example. For example, the receiver RF coil portion 201a and the receiver RF coil portion 201b may have, as the decoupling structure, only one of: the structure with the parts of the adjacent coil elements overlapping each other; and the decoupling circuitry arranged between the adjacent coil elements.

Furthermore, in another example, the plural coil elements 231a included in the receiver RF coil portion 201a and the plural coil elements 231b included in the receiver RF coil portion 201b may be arranged such that one of the coil elements included in one of these receiver RF coil portions overlaps part of two of the coil elements included in the other one of the receiver RF coil portions.

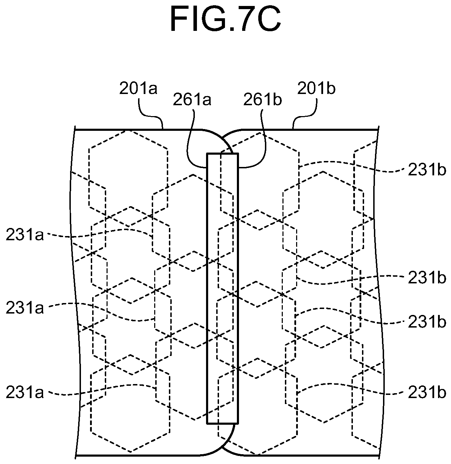

For example, as illustrated in FIG. 7C, the plural coil elements 231a included in the receiver RF coil portion 201a and the plural coil elements 231b included in the receiver RF coil portion 201b are arranged to be positionally shifted from each other by a distance that is half a length of one coil element in the longitudinal direction of the couchtop 115.

Between the plural coil elements 231a included in the receiver RF coil portion 201a and the plural coil elements 231b included in the receiver RF coil portion 201b, the above described configuration results in a partial overlap between all of adjacent ones of the coil elements when the end portion of the receiver RF coil portion 201a and the end portion of the receiver RF coil portion 201b have been connected to each other and thus enables even more efficient decoupling without use of the decoupling circuitry.

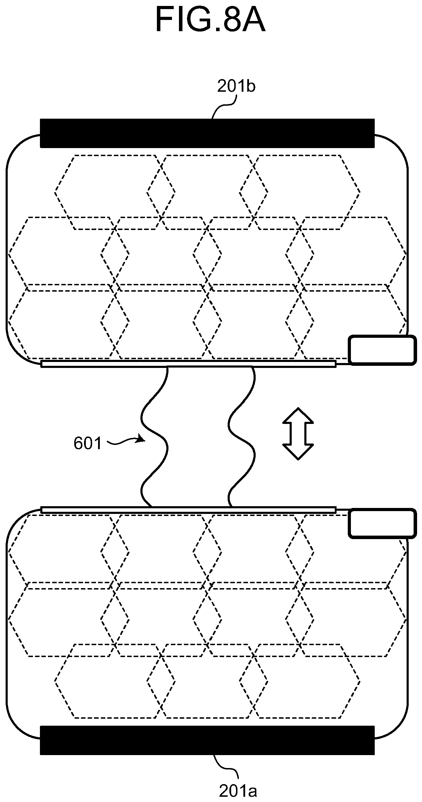

FIG. 8A to FIG. 8C are diagrams illustrating examples of the coil holding member 204 included in the body receiver RF coil according to the first embodiment.

For example, as illustrated in FIG. 8A, the coil holding member 204 is implemented by connecting between the two receiver RF coil portions 201a and 201b of the body receiver RF coil by an elastic member 601 including a material having elasticity, such as gum.

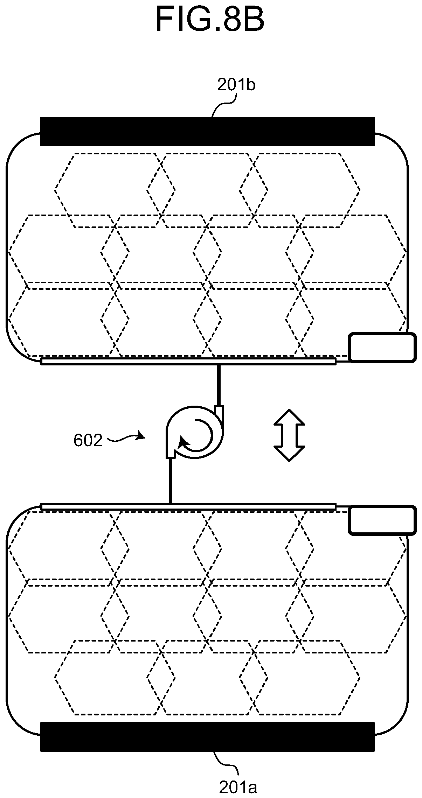

Or, for example, as illustrated in FIG. 8B, the coil holding member 204 may be implemented by connecting between the two receiver RF coil portions 201a and 201b of the body receiver RF coil by a winding mechanism 602.

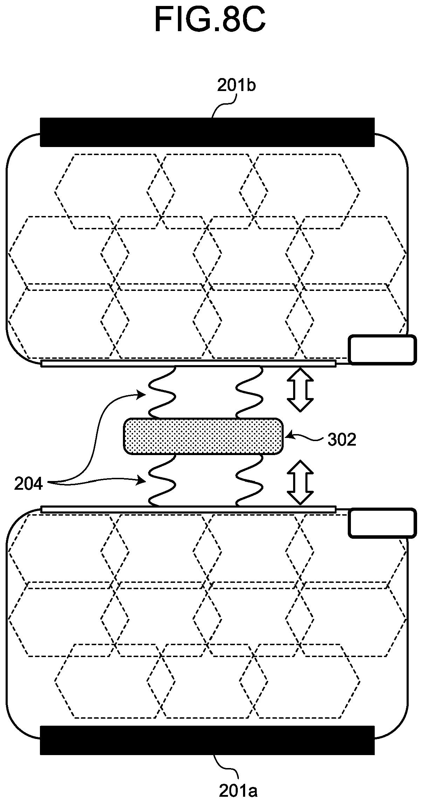

Or, for example, as illustrated in FIG. 8C, the coil holding member 204 may include two coil holding members and may be implemented by one of the coil holding members connecting between the receiver RF coil portion 201a of the body receiver RF coil and a couchtop supporting portion 302 and the other one of the coil holding members connecting between the receiver RF coil portion 201b of the body receiver RF coil and the couchtop supporting portion 302.

As described above, in the first embodiment, the couchtop 115 of the couch 111 has the space to stow the body receiver RF coils in the side portions and bottom portion of the couchtop 115. The body receiver RF coils are configured to be used by being pulled out and/or pushed out from the side portions and bottom portion of the couchtop 115.

This configuration enables: stowage of the body receiver RF coils, which are to be placed on the subject 114 when imaging of the body portion is performed, into the side portions and bottom portion of the couchtop 115; and placement of the body receiver RF coils pulled out and/or pushed out when needed.

Therefore, the first embodiment enables placement of the body receiver RF coils on the subject 114 to be facilitated.

In the first embodiment, the couchtop 115 has the couchtop frame 203 that supports a load on the couchtop 115 and that is movable to the imaging region. The couchtop frame 203 has the upper surface where the subject 114 is to be placed on and the couchtop frame 203 forms the space to stow the body receiver RF coils in the side portions and bottom portion of the couchtop 115.

This configuration enables the couchtop 115 to have enough strength to endure the load including that from the subject 114 even in the case where the space to stow the body receiver RF coils has been provided in the couchtop 115.

The above described MRI apparatus 100 according to the first embodiment may be implemented by modification of the configurations of the couchtop 115 and body receiver RF coils as appropriate. Modified examples related to the first embodiment will thus be described hereinafter as other embodiments. Any description that is the same as that of the first embodiment will be omitted and features different from those of the first embodiment will mainly be described with respect to the following embodiments.

### Second Embodiment

For example, an extensible and contractible receiver RF coil may be used as a body receiver RF coil. An example of such a case will be described hereinafter as a second embodiment.

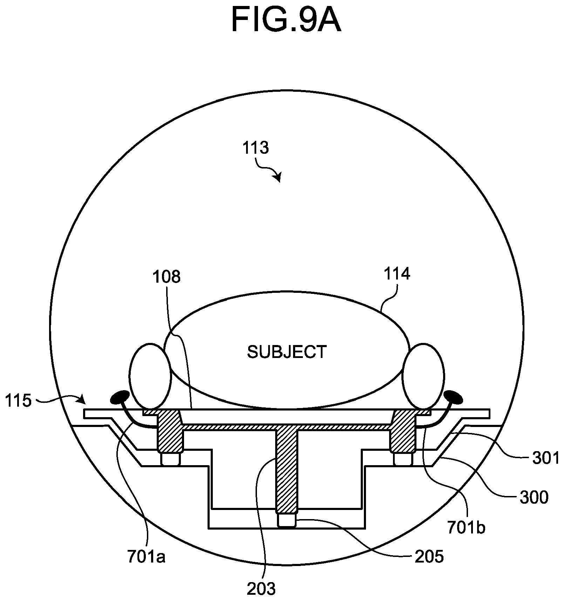

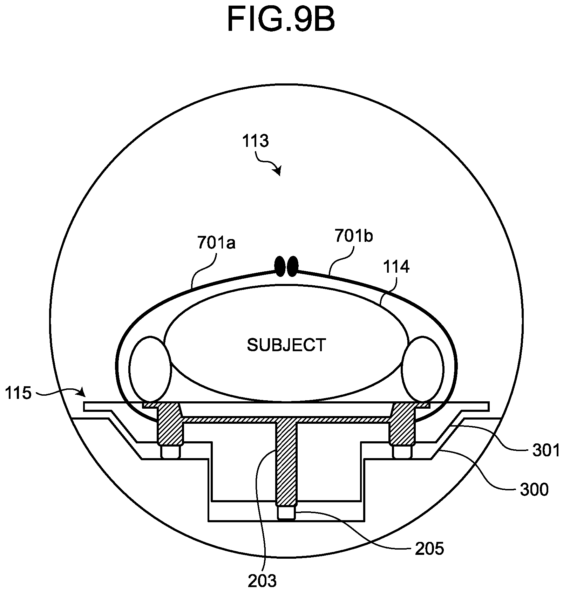

FIG. 9A and FIG. 9B are sectional views of an example of a couchtop 115 and a body receiver RF coil, according to the second embodiment. Specifically, FIG. 9A illustrates a state where the coil of the example of the couchtop 115 and body receiver RF coil according to the second embodiment has been stowed, and FIG. 9B illustrates a state where the coil of the example of the couchtop 115 and body receiver RF coil according to the second embodiment has been pulled out and/or pushed out.

For example, as illustrated in FIG. 9A, in this embodiment, the body receiver RF coil is an extensible and contractible receiver RF coil and includes two receiver RF coil portions 701a and 701b. For example, an extensible and contractible receiver RF coil disclosed in Patent Literature 4 may be used as the body receiver RF coil according to this embodiment.

One of end portions of each of the two receiver RF coil portions 701a and 701b is connected to a couchtop frame 203, and when the body receiver RF coil is not being used, the two receiver RF coil portions 701a and 701b are stowed in a space formed in side portions of the couchtop 115 by a couchtop frame 203 in a couchtop bottom cover 301.

For example, as illustrated in FIG. 9B, when the body receiver RF coil is used in imaging, the body receiver RF coil is placed on a subject 114 by being pulled out and/or pushed out from the side portions of the couchtop 115. The body receiver RF coil is configured such that the two receiver RF coil portions 701a and 701b are able to be connected to each other on the subject 114 by extending.

That is, in this embodiment, the couchtop 115 of a couch 111 has the space to stow the body receiver RF coil in the side portions of the couchtop 115. Specifically, the couchtop frame 203 forms the space to stow the body receiver RF coil in the side portions of the couchtop 115. The body receiver RF coil is configured to be used by being pulled out and/or pushed out from the side portions of the couchtop 115.

FIG. 9A and FIG. 9B illustrate the example where the space has been formed in each of the side portions and a bottom portion of the couchtop 115 by the couchtop frame 203, similarly to the first embodiment, but in this embodiment, the space may be not formed in the bottom portion of the couchtop 115.

As described above, in this second embodiment, the body receiver RF coil is configured to be extensible and contractible.

This configuration enables reduction in the space to stow the body receiver RF coil in the couchtop 115 and enables the couchtop 115 to have more strength.

### Other Embodiments

With respect to the second embodiment, an example of the case where an extensible and contractible receiver RF coil is used as the body receiver RF coil has been described above, but the second embodiment is not limited to this example.
For example, a windable receiver RF coil may be used as the body receiver RF coil.

For example, the body receiver RF coil is a windable receiver RF coil having flexibility and includes two receiver RF coil portions 701a and 701b having flexibility.

One of end portions of each of the two receiver RF coil portions 701a and 701b is connected to a winding mechanism arranged in the space formed in the side portions of the couchtop 115 by the couchtop frame 203 in the couchtop bottom cover 301, and when the body receiver RF coil is not being used, the two receiver RF coil portions 701a and 701b are stowed in the space in the side portions of the couchtop 115 by being wound by the winding mechanism.

When the body receiver RF coil is used in imaging, the body receiver RF coil is placed on the subject 114 by being pulled out and/or pushed out from the side portions of the couchtop 115. The body receiver RF coil is configured such that the two receiver RF coil portions 701a and 701b are able to be connected to each other on the subject 114 by being pulled out and/or pushed out from the winding mechanism.

Similarly to the second embodiment, this configuration enables reduction in the space to stow the body receiver RF coil in the couchtop 115 and enables the couchtop 115 to have more strength.

Furthermore, in the examples of the case described with respect to the above described embodiments, the couchtop 115 of the couch 111 has the space to stow the body receiver RF coils in the widthwise side portions and bottom portion of the couchtop 115, but the embodiments are not limited to these examples. For example, the couchtop 115 of the couch 111 may have a space to stow receiver coils at lengthwise end portions of the couchtop 115 in addition to that in the widthwise side portions of the couchtop 115 or instead of that in the widthwise side portions of the couchtop 115. The receiver coils are, for example, a head receiver RF coil to receive MR signals mainly from the head of a subject and a leg receiver RF coil to receive MR signals mainly from the legs of the subject. In this case, the receiver coils are each configured to be placed on the subject 114 by being pulled out and/or pushed out from at least one of the lengthwise end portions of the couchtop 115.

Generally, many head receiver RF coils are larger in weight than receiver coils for other regions and much manpower is needed for carrying them and placing them on subjects. Therefore, stowing the head receiver RF coil in the space provided in the lengthwise end portions of the couchtop 115 and enabling the head receiver RF coil to be placed by being pulled out and/or pushed out when needed, like in the above described configuration, facilitate placement of the head receiver RF coil on the subject 114.

Furthermore, a space may be not provided in the widthwise side portions and lengthwise end portions of the couchtop 115 and a space may be provided only in the bottom portion of the couchtop 115. In this case, receiver coils are each configured to be placed on the subject 114 by being pulled out and/or pushed out from the bottom portion of the couchtop 115 and passing outside the side portions or end portions of the couchtop 115.

Furthermore, in the example of the case described above with respect to the embodiments, the couchtop 115 has the space to stow the body receiver RF coil in both of the widthwise side portions of the couchtop 115, the body receiver RF coil has the two receiver RF coil portions 201a and 201b, and these receiver RF coil portions 201a and 201b are configured to be placed on the subject 114 by being respectively pulled out and/or pushed out from the one and the other one of the widthwise side portions of the couchtop 115, but the embodiments are not limited to this example.

For example, the couchtop 115 may have a space to stow a receiver coil in one of the widthwise side portions of the couchtop 115 and the receiver coil may be configured to be pulled out and/or pushed out from the one of the widthwise side portions of the couchtop 115 and connected and fixed to the other one of the widthwise side portions of the couchtop 115.

Or the couchtop 115 may have a space to stow a receiver coil in one of the lengthwise end portions of the couchtop 115 and the receiver coil may be configured to be pulled out and/or pushed out from the one of the lengthwise end portions of the couchtop 115 and connected and fixed to the other one of the lengthwise end portions of the couchtop 115.

Furthermore, in the above described embodiments, the receiver coil is placed on the subject 114 by being pulled out and/or pushed out from the couchtop 115, but the receiver coil may be pulled out and/or pushed out by an operator holding and moving part of the receiver coil, the operator being an operator to place the receiver coil, or by use of a pulling mechanism or pushing mechanism provided in the couchtop 115.

For example, as an example of the pulling mechanism for pulling out the receiver coil, the couchtop 115 includes a rotation unit provided at a widthwise side portion of the couchtop 115 or a lengthwise end portion of the couchtop 115, a rotation drive unit that rotates the rotation unit in one direction or a reverse direction, and a pulling control unit. The rotation unit is implemented by a roller, for example, and moves the receiver coil to outside of the couchtop 115 from the space in the couchtop 115 by rotating in the one direction in a state of being in contact with the receiver coil. Furthermore, the rotation unit moves the receiver coil from the outside of the couchtop 115 to a space in the couchtop 115 by rotating in the reverse direction in the state of being in contact with the receiver coil. The pulling control unit receives an instruction to pull out the receiver coil, from the operator via an operating unit, such as a button, controls the rotation drive unit to rotate the rotation unit in the one direction according to the instruction, and thereby pulls out the receiver coil stowed in the space in the couchtop 115 to the outside of the couchtop 115. Furthermore, the pulling control unit receives an instruction to stow the receiver coil, from the operator via an operating unit, such as a button, controls the rotation drive unit to rotate the rotation unit in the reverse direction according to the instruction, and thereby stows the receiver coil, which has been pulled out to the outside of the couchtop 115, into the space in the couchtop 115.

Furthermore, the couchtop 115 includes, as an example of the pushing mechanism for pushing out the receiver coil, a biasing unit, a holding unit, and a pushing control unit that have been provided inside the couchtop 115. The biasing unit is implemented by a spring, for example, and biases the receiver coil stowed in a space in the couchtop 115 in a direction of moving the receiver coil to outside of the couchtop 115. The holding unit holds the receiver coil in the space in the couchtop 115 against biasing force of the biasing unit. The pushing control unit receives an instruction to push out the receiver coil, from the operator via an operating unit, such as a button, releases the receiver coil held by the holding unit according to the instruction, and thereby pushes out the receiver coil stowed in the space in the couchtop 115 to the outside of the couchtop 115.

The components of each device/apparatus according to the embodiments described above have been functionally and conceptually illustrated in the drawings and are not necessarily configured physically as illustrated in the drawings. That is, specific modes of distribution and integration of each device/apparatus are not limited to those illustrated in the drawings, and all or part of each device/apparatus may be configured to be distributed or integrated functionally or physically in any units, according to various loads and/or use situations, for example. In addition, all or any part of the processing functions executed at each device/apparatus may be implemented by a CPU and programs analyzed and executed by the CPU, or implemented as hardware by wired logic.

Furthermore, of the processing described above with respect to the embodiments, all or part of any processing described as being performed automatically may be performed manually, or all or part of any processing described as being performed manually may be performed automatically by a publicly known method. In addition, the processing procedures, control procedures, specific names, and information including various data and parameters, which have been described above and illustrated in the drawings, may be modified in any way except stated otherwise specifically.

At least one of the embodiments described above enables placement of receiver coils on subjects to be facilitated.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

## Claims

1. A magnetic resonance imaging apparatus (100), comprising:
a receiver coil (106, 107) that receives a magnetic resonance signal; and
a couchtop (115) that a subject is placed on and that moves the subject to an imaging region, wherein
the couchtop (115) has a space to stow the receiver coil (106, 107) in one or more portions of: widthwise side portions of the couchtop (115); lengthwise end portions of the couchtop (115); and a bottom portion of the couchtop (115), and
the receiver coil is configured to be used by being pulled out and/or pushed out from the one or more portions of the couchtop (115).

2. The magnetic resonance imaging apparatus (100) according to claim 1, wherein
the couchtop (115) has a couchtop frame (203) that supports a load on the couchtop (115) and that is movable to the imaging region, and
the couchtop frame (203) has an upper surface where the subject is to be placed on and the couchtop frame (203) forms the space in the one or more portions of the couchtop (115).

3. The magnetic resonance imaging apparatus (100) according to claim 2, wherein
the couchtop frame (203) has a couchtop supporting portion (302, 302a-h) that supports the upper surface, and
the couchtop supporting portion (302, 302a-h) has a column-shaped or wall-shaped structure.

4. The magnetic resonance imaging apparatus (100) according to any one of claims 1 to 3, wherein the receiver coil (106, 107) is a body receiver coil that receives a magnetic resonance signal from a body portion of the subject.

5. The magnetic resonance imaging apparatus (100) according to any one of claims 1 to 3, wherein the receiver coil (106, 107) is a head receiver coil that receives a magnetic resonance signal from the head of the subject.

6. The magnetic resonance imaging apparatus (100) according to any one of claims 1 to 3, wherein the receiver coil (106, 107) is configured to be placed on the subject by being pulled out and/or pushed out from at least one of the widthwise side portions of the couchtop (115) or at least one of the lengthwise end portions of the couchtop (115).

7. The magnetic resonance imaging apparatus (100) according to any one of claims 1 to 3, wherein the space accommodates a plurality of the receiver coils arranged to be lined up in a direction of movement of the couchtop (115).

8. The magnetic resonance imaging apparatus (100) according to any one of claims 1 to 3, further comprising, separately from the receiver coil (106, 107) to be stowed in the space, a spine receiver coil that receives a magnetic resonance signal from the back of the subject.

9. The magnetic resonance imaging apparatus (100) according to any one of claims 1 to 3, wherein
the couchtop (115) has the space to stow the receiver coil (106, 107) in both of the widthwise side portions of the couchtop (115), and
the receiver coil (106, 107) has a first receiver coil portion (201a, 220a, 221a, 222a, 701a) and a second receiver coil portion (201b, 220b, 221b, 222b, 701b), the first receiver coil portion (201a, 220a, 221a, 222a, 701a) being configured to be placed on the subject by being pulled out and/or pushed out from one of the widthwise side portions of the couchtop (115), the second receiver coil portion (201b, 220b, 221b, 222b, 701b) being configured to be placed on the subject by being pulled out and/or pushed out from the other one of the widthwise side portions of the couchtop (115).

10. The magnetic resonance imaging apparatus (100) according to claim 9, wherein the first receiver coil portion (201a, 220a, 221a, 222a, 701a) and the second receiver coil portion (201b, 220b, 221b, 222b, 701b) are configured such that an end portion of the first receiver coil portion and an end portion of the second receiver coil portion are connectible to each other on the subject.

11. The magnetic resonance imaging apparatus (100) according to claim 10, wherein
the first receiver coil portion (201a, 220a, 221a, 222a, 701a) includes a first coil element (231a) arranged near the end portion to be connected to the second receiver coil portion (201b, 220b, 221b, 222b, 701b),
the second receiver coil portion (201b, 220b, 221b, 222b, 701b) includes a second coil element (231b) arranged near the end portion to be connected to the first receiver coil portion (201a, 220a, 221a, 222a, 701a), and
at least one of the first receiver coil portion (201a, 220a, 221a, 222a, 701a) and the second receiver coil portion (201b, 220b, 221b, 222b, 701b) has a decoupling structure that implements decoupling between the first coil element (231a) and the second coil element (231b) when the end portion of the first receiver coil portion (201a, 220a, 221a, 222a, 701a) and the end portion of the second receiver coil portion (201b, 220b, 221b, 222b, 701b) are connected to each other.

12. The magnetic resonance imaging apparatus (100) according to claim 11, wherein at least one of the first receiver coil portion (201a, 220a, 221a, 222a, 701a) and the second receiver coil portion (201b, 220b, 221b, 222b, 701b) has, as the decoupling structure: a structure having part of the first coil element (231a) and part of the second coil element (231b) overlapping each other when the end portion of the first receiver coil portion (201a, 220a, 221a, 222a, 701a) and the end portion of the second receiver coil portion (201b, 220b, 221b, 222b, 701b) are connected to each other; and/or decoupling circuitry that is arranged between the first coil element (231a) and the second coil element (231b) when the end portion of the first receiver coil portion (201a, 220a, 221a, 222a, 701a) and the end portion of the second receiver coil portion (201b, 220b, 221b, 222b, 701b) are connected to each other.

13. The magnetic resonance imaging apparatus (100) according to claim 11, wherein at least one of the first receiver coil portion (201a, 220a, 221a, 222a, 701a) and the second receiver coil portion (201b, 220b, 221b, 222b, 701b) has a positioning structure that positions a connection position of the end portions of the first and second receiver coil portions to a position where decoupling by the decoupling structure is possible.

14. The magnetic resonance imaging apparatus (100) according to claim 13, wherein at least one of the first receiver coil portion (201a, 220a, 221a, 222a, 701a) and the second receiver coil portion (201b, 220b, 221b, 222b, 701b) has, as the positioning structure, a non-magnetic fixation tool attached to the end portion of the at least one of the first receiver coil portion (201a, 220a, 221a, 222a, 701a) and the second receiver coil portion (201b, 220b, 221b, 222b, 701b).

15. The magnetic resonance imaging apparatus (100) according to any one of claims 1 to 3, wherein
the couchtop (115) has the space to stow the receiver coil in one of the widthwise side portions of the couchtop (115) or one of the lengthwise end portions of the couchtop (115), and
the receiver coil is configured to be pulled out and/or pushed out from the one of the widthwise side portions of the couchtop (115) or the one of the lengthwise end portions of the couchtop (115) and to be connected and fixed to the other one of the widthwise side portions of the couchtop (115) or the other one of the lengthwise end portions of the couchtop (115) .
